# EUROPEAN PATENT APPLICATION

(11) **EP 1 776 979 A1**
(43) Date of publication of application: **25.04.2007**
(21) Application number: 06255368.0
(22) Date of filing: 18.10.2006
(51) Int. Cl.: A61M 25/00

(54) **Flexible metallic cannula infusion set**

(30) Priority: 19.10.2005 US 728123 P
(71) Applicant: Animas Corporation, West Chester PA 19380 (US)
(72) Inventor: DeStefano, Mark, Collegeville, PA 19426 (US)
(74) Representative: Tunstall, Christopher Stephen

(57) **Abstract**

A cannula housing assembly for an infusion set (100,200,300) is disclosed. The cannula housing assembly includes a cannula housing (102) and a cannula (110,210) extending distally from the cannula housing. The cannula may be constructed from a shape memory alloy or a superelastic alloy.

## Description

### CROSS REFERENCE TO RELATED APPLICATION

The present application claims priority from U.S. Provisional Patent Application Serial No. 60/728,123, filed on October 19, 2005.

### FIELD OF THE INVENTION

The present invention relates to an infusion set that incorporates a flexible metal cannula that is constructed from either a shape memory alloy or a superelastic alloy.

### BACKGROUND OF THE INVENTION

Frequent or continuous subcutaneous and intravenous injection of medication is often accomplished through the use of an infusion set or injection port, which may remain in place for up to several days. In the case of frequent injections, this reduces the need to constantly puncture the skin, reducing the chance of infection and the formation of scar tissue. For continuous subcutaneous delivery of medication such as commonly used with portable insulin pumps, an infusion set is often used to provide a method of temporarily detaching the pump and fluid line for activities such as dressing or bathing.

Conventional infusion sets, fluid delivery systems, or catheters where fluid enters the body have commonly been constructed in 2 ways. In the early designs, which are still in use, a small rigid stainless steel hypodermic needle is attached to the ends of flexible tubing at a hub or housing for insertion into the body. These needles are very stiff and rigid but can be made sharp enough to pierce the skin. Once inserted though, the rigidity of these needles not only made them painful but also significantly limited the movement of the patient's body in the area of the needle. Also, needles remaining under the skin for long periods of time would continually pinch any tissue that was moving in relation to the needle and cause pain. These needles could also tear the tissue and veins if too much relative movement occurred near the tip. In addition, the stainless steel tube wall thickness could not be made as thin as possible because with very thin walls, the stainless steel tube is also easy to damage, collapse, kink, and pinch off. Even with traditional wall thickness stainless steel, needles are rigid and are prone to kink and be permanently damaged rather then flex and bend with the patient's movement. This thickness is in the range of about 0.102 mm (0.004 inches). Thus, needles made of this material, although small, are still large enough to cause pain and discomfort to the user and still require a relatively larger wall thickness to avoid bending.

To overcome the inflexibility and reduce discomfort, polymer cannulae have been developed. In this design, a softer polymer cannula replaces the metal needle tip. Since this cannula is soft and cannot pierce the skin, a solid metal trocar needle is inserted through the cannula for the purpose of piercing the skin and introducing the cannula below the surface of the skin. Once the cannula is below the skin surface at the desired depth, the metal trocar is removed and only the soft cannula remains under the skin. Compared to the stainless steel cannula, this greatly reduces pain, and the flexibility of the polymer allows the tissues to flex and move with little discomfort. However, polymer cannulae have drawbacks. They can be easily crimped, flattened, kinked, pinched, and damaged during insertion and bending. Additionally, polymer cannulae can be displaced laterally, causing pinching and closure of the fluid path, and the polymers can elicit an allergic reaction from surrounding tissues. Further, the polymer cannulae also complicate the device assembly processes because the polymers are typically hard to assemble and bond in place or hold in place with high mechanical strength and without mechanical damage. The polymer cannulae must also be made with a thicker wall than a metal tube to remain circular and not collapse or pinch off when installed. This thickness is in the range of 0.076 to 0.127 mm (0.003 to 0.005 inches).

Accordingly, a need exists to develop a cannula that will minimize pain inflicted upon the patient, yet be able to withstand kinking and collapsing forces after insertion.

### SUMMARY OF THE INVENTION

Briefly, the present invention provides a cannula housing assembly for an infusion set. The cannula housing assembly includes a cannula housing and a cannula extending distally from the cannula housing. The cannula is constructed from a shape memory alloy, wherein the cannula is constructed from one of a superelastic material and a shape memory material.

Additionally, the present invention provides a cannula housing assembly for an infusion set. The cannula housing assembly includes a base and a cannula housing coupled to the base. A cannula extends distally from the cannula housing. The cannula is constructed from one of a superelastic material and a shape memory material.

Further, the present invention provides a method of inserting a cannula into a patient. The method comprises the steps of: providing a cannula from a shape memory material; forming the cannula into a desired shape; reforming the cannula into an insertion shape; inserting the cannula into the patient; and allowing body heat from the patient to revert the cannula from the insertion shape to the desired shape.

### BRIEF DESCRIPTION OF THE DRAWINGS

The foregoing summary, as well as the following detailed description a desired embodiments of the invention, will be better understood when read in conjunction with the appended drawings, which are incorporated herein and constitute part of this specification. For the purposes of illustrating the invention, there are shown in the drawings exemplary embodiments of the invention. It should be understood, however, that the invention is not limited to the precise arrangements and instrumentalities shown. In the drawings, the same reference numerals are employed for designating the same elements throughout the several figures. In the drawings:
Fig. 1a is a side elevational view showing a superelastic cannula according to an exemplary embodiment of the present invention in a non-stressed state;
Fig. 1b is a side elevational view showing the superelastic cannula of Fig. 1a, with a force applied to a free end of the cannula;
Fig. 1c is a side elevational view of the cannula of Fig. 1b, with the force having been removed from the cannula;
Fig. 2 is a side elevational view, partially in section, of an exemplary infusion set using the cannula of Fig. 1a being inserted into a patient;
Fig. 3 is a side elevational view, partially in section, of an exemplary infusion set using the cannula of Fig. 1a having been inserted into a patient;
Fig. 4 is a top plan view of the infusion set of Fig. 3, demonstrating the flexibility of the cannula in the patient;
Fig. 5a is a side elevational view showing a shape memory cannula according to another exemplary embodiment of the present invention in a pre-formed state;
Fig. 5b is a side elevational view of the shape memory cannula of Fig. 5a in a deformed state;
Fig. 5c is a side elevational view of the shape memory cannula of Fig. 5b, with heat being applied to the cannula;
Fig. 5d is a side elevational view of the shape memory cannula of Fig. 5c, having reverted back to its pre-formed state;
Fig. 6 is a side elevational view, partially in section, of an exemplary infusion set using the cannula of Fig. 5a being inserted into a patient;
Fig. 7 is a perspective view of the infusion set of Fig. 6 using the cannula of Fig. 5a being inserted into the patient;
Fig. 8 is a side elevational view, partially in section, of the infusion set using the cannula of Fig. 5a being inserted into the patient;
Fig. 9 is a bottom perspective view of an infusion set, using the cannula of Figs. 1a-1c with the infusion set housing of Fig. 8, having been inserted into a patient; and
Fig. 10 is a sectional view of the infusion set of Fig. 9, having been inserted into a patient and translated longitudinally relative to the patient.

### DETAILED DESCRIPTION OF THE INVENTION

Certain terminology is used herein for convenience only and is not to be taken as a limitation on the present invention. The terminology includes the words specifically mentioned, derivatives thereof and words of similar import. As used herein, the term "superelastic" is defined as the ability of a structure deformed under a load to return to its original configuration upon the removal of the load. The term "shape memory alloy" is defined as a metal made from the combination of two or more metals that exhibit hardness and elasticity properties that change radically at distinct temperatures. The term "distal" is defined to mean a direction closer to the insertion tip of a cannula described herein and "proximal" is defined to mean a direction farther from the insertion tip of the cannula described herein. The following describes exemplary embodiments of the invention. However, it should be understood based on this disclosure, that the invention is not limited by the exemplary embodiments of the invention.

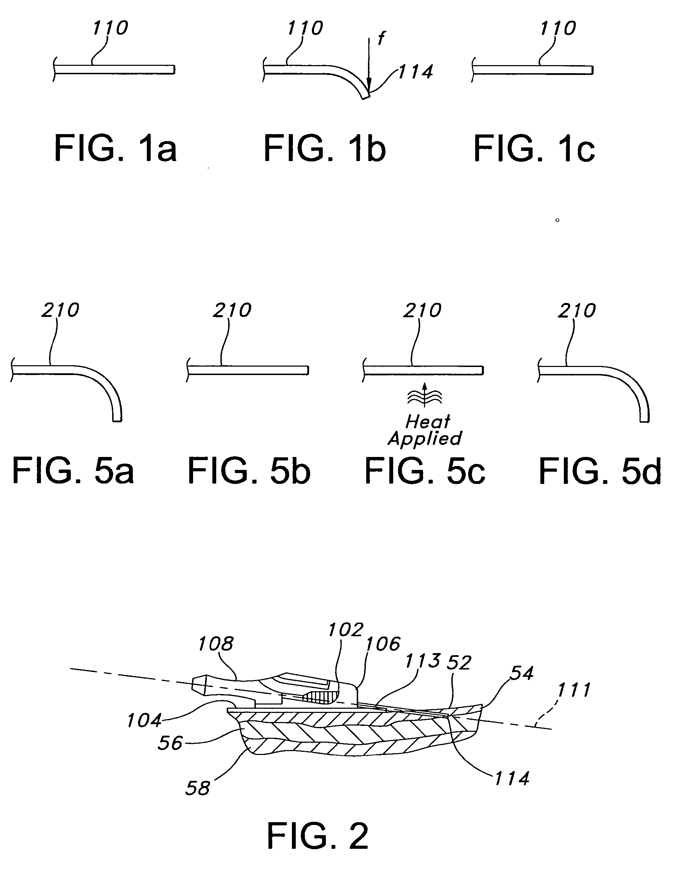

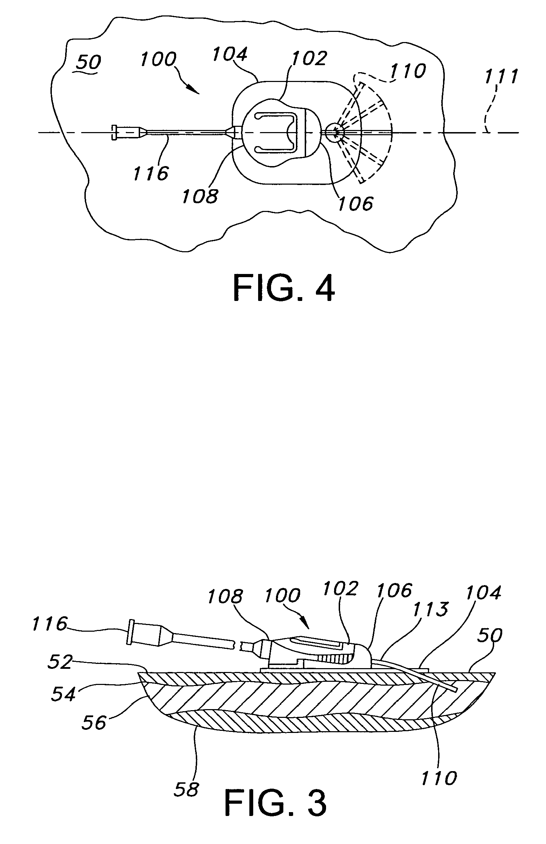

Referring generally to the figures, infusion sets 100, 200, 300 according to the present invention are each characterized by an extremely flexible metallic cannula 110 (see Figs. 1a-1c), 210 (see Figs. 2a-2c) formed of Nitinol material that, when inserted, remains flexible and provides the beneficial attributes of both metallic and soft polymeric cannulae without the drawbacks of such cannulae.

The cannulae 110, 210 are desirably constructed from Nitinol, which is a metallic alloy consisting essentially of nickel and titanium. Typically, Nitinol includes about 55% by weight of nickel and 45% by weight of titanium. Other materials may be added to "fine tune" the properties of the Nitinol. Nitinol exhibits two unique behaviors, depending on its processing. The two behaviors are superelasticity and shape memory, which are described below.

Nitinol is generally provided in one of two types of crystal structures: martensite, which is finished at a low temperature, and austenite, which is finished at a higher temperature. Martensite in Nitinol can be stress induced if stress is applied in the temperature range slightly above the austenite finish transformation temperature. Less energy is needed to stress induce and deform martensite in this state than to deform austenite by conventional means. Because the martensite has been formed above its normal temperature, the martensite can be easily deformed by a small amount of force and reverts immediately, or "springs back", to the un-deformed austenite, its original shape, once the force is removed. This process provides a very springy, rubber-like elasticity in the Nitinol. This characteristic exists because austenite is the stable phase at this temperature under no load conditions. Therefore, a Nitinol tube in the superelastic state can be flexed to a very high degree without damage and once the stress is removed, the tube will revert to its original shape. Nitinol is superelastic in a temperature range of about 50 degrees Centigrade above the austenite finish temperature. Nitinol is at its optimum superelastic behavior at body temperature (about 37 degrees Centigrade).

In addition to being a superelastic alloy, Nitinol is also a shape memory alloy. A shape memory alloy (SMA) is a metal that remembers its geometry. After the alloy is deformed, it regains its original geometry by itself during heating (one-way effect) or, at higher ambient temperatures, simply during unloading (pseudo-elasticity). These extraordinary properties are due to a temperature-dependent martensitic phase transformation from a low-symmetry to a highly symmetric crystallographic structure.

When Nitinol is in its martensite form, below the transformation temperature, the Nitinol alloy can be easily deformed and is malleable. Once deformed, the Nitinol alloy will remain in the deformed state at room temperature. When the alloy is then heated above the transformation temperature, the alloy reverts to austenite and recovers its previous original shape with high force. As the alloy cools, the alloy reverts again back to the martensite form, exhibiting the shape memory property of the Nitinol.

The transformation from austenite to martensite (cooling) and the reverse cycle (heating) does not occur at the same temperature and the temperature of the transformation can be controlled by the thermo-mechanical processing history of the specific alloy. The shape memory effect is repeatable for many cycles. Alloys are available with transformation temperatures between 45 and 120 degrees Centigrade. However, the most interesting shape memory alloys for application in an infusion set is the alloy with a body temperature transformation at 37 degrees Centigrade. In this condition, the deformed alloy will recover its original shape and become and remain super elastic when placed inside the human body while remaining at 37 degrees Centigrade.

In a superelastic state, the Nitinol comprising cannula 110 allows cannula 110 to be deformed under a load, but to revert to its pre-load condition after removal of the load. Figs. 1a-1c demonstrate the property of the superelastic Nitinol. Fig. 1a shows cannula 110 in a "no load" condition. In Fig. 1b, a force "f" is applied to a free end 114 of cannula 110, deflecting free end 114. It should be noted that force "f" is relatively low, such as less than about 0.3 grams, but free end 114 easily deflects. In this condition, no buckling of cannula 110 occurs. In Fig. 1c, the force "f" has been removed and cannula 110 has returned to its original position as shown in Fig. 1a.

As an example of Nitinol's superelastic property, seen in Figs. 2-4, infusion set 100 is shown. Infusion set 100 may be an infusion set as described in U.S. Patent No. 6,572,586, which is incorporated herein by reference as through fully set forth herein. Alternatively, infusion set 100 may be an infusion set as described in U.S. Provisional Patent Application Serial No. 60/728,124 (Attorney Docket No. ANMS-200USP), filed October 19, 2005.

A side view of infusion set 100 inserted into a patient 50 is shown in Fig. 2. Infusion set 100 includes a housing 102 fixedly mounted to an adhesive pad 104. Housing 102 includes a base, an inserter (for initial insertion into the patient) and a tubing assembly for fluid transfer. Adhesive pad 104 secures infusion set 100 to the epidermal skin layer 52 of patient 50. Housing 102 also includes a distal end 106 and a proximal end 108. Flexible cannula 110 extends from distal end 106 of housing 102. A longitudinal axis 111 extends along cannula 110. A lumen 113 extends through cannula 110 to provide fluid flow communication through cannula 110. Cannula 110 may be inserted by using an introducer 114, such as a metal insertion needle or trocar, that is initially inserted through lumen 113 and extends distally beyond cannula 110.

Cannula 110 and introducer 114 are inserted subcutaneously through epidermal skin layer 52, the dermal skin layer 54, and into the fat tissue layer 56. It is important to note that cannula 110 should not be inserted into the muscle layer 58 below fat tissue layer 56. After insertion of cannula 110 into patient 50, introducer 114 is then removed from lumen 113 by sliding introducer 114 proximally through lumen 113. Optionally, introducer 114 may also be constructed from Nitinol to provide flexibility during insertion, if so desired. With introducer 114, cannula 110 may have a wall thickness as thin as about 0.025 mm (0.001 in) in order to obtain maximum flexibility.

Alternatively, cannula 110 may be provided with a sharpened tip and introducer 114 may be omitted. If introducer 114 is used, after insertion of cannula 110, introducer 114 is removed from cannula 110 according to known methods and a tubing assembly 116 is coupled to proximal end 108 of housing 102. Fig. 3 shows tubing assembly 116 coupled to housing 102.

In use, medication, such as insulin, is pumped through tubing assembly 116 to infusion set 100, and through cannula 110. The medication is then administered to patient 50, where the medication is absorbed by the patient.

Fig. 4 shows how cannula 110 can be deflected to the left and right of longitudinal axis 111. Similarly, those skilled in the art will recognize that cannula 110 can be deflected above and below longitudinal axis 111. Cannula 110 is free to flex with tissue movement and return to the original position.

A cannula 210 exhibiting shape memory properties is shown in Figs. 5a-5d. Initially, with the Nitinol in its austenitic state, cannula 210 is "pre-formed" with the bend shown in Fig. 5a. Cannula 210 is cooled to the Nitinol's martensitic state, where cannula 210 is "captured" in the bent shape. Cannula 210 is then shaped into a straight cannula 210, shown in Fig. 5b, for insertion into a patient. After insertion into the patient, cannula 210 is heated back to its austenitic temperature, such as by body heat, as shown in Fig. 5c. When cannula 210 reaches its austenitic temperature, cannula 210 snaps back to its pre-formed shape of Fig. 5a, as shown in Fig. 5d.

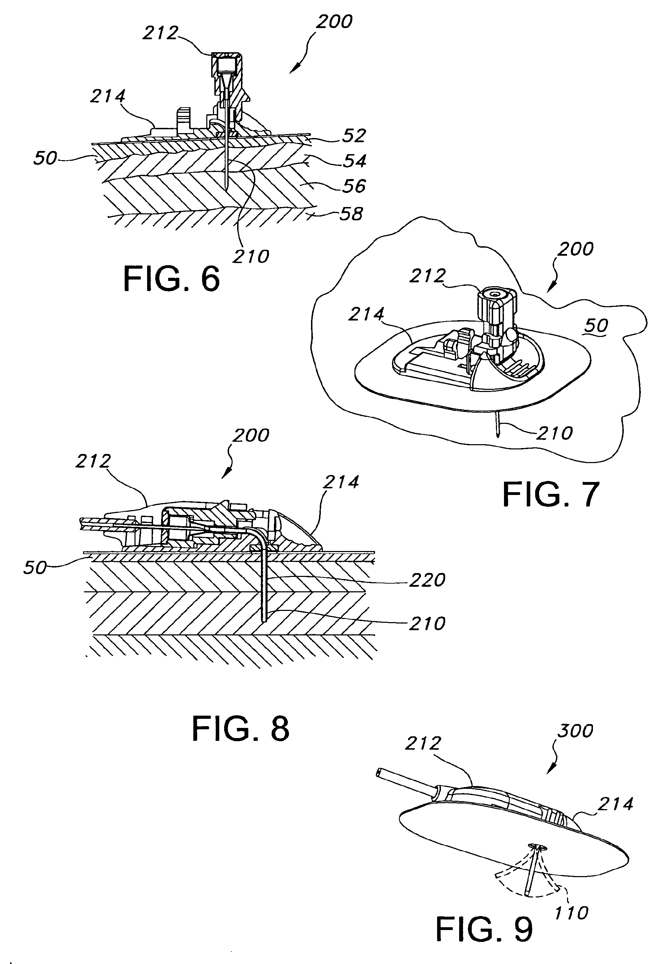

As seen in Figs. 6 and 7, insertion set 200 includes a cannula housing 212 that is pivotally mounted on a base 214 such that cannula 210 is inserted into a patient 50 at an angle substantially normal to a plane of the patient 50. Cannula 210 is in its shape memory state, having been manufactured according to the process described above and depicted in Figs. 5a-5b. Cannula 210 has been deformed from the configuration shown in Fig. 5a to the configuration shown in Fig. 5b.

After cannula 210 has been in patient 50 sufficiently long to heat cannula 210 to body temperature (about 37 degrees Centigrade), cannula housing 212 may be pivoted about base 214 to the position shown in Fig. 8. As can be seen, cannula 210 bends back to its pre-formed shape as depicted in Fig. 5d.

While infusion set 200 shown in Figs. 6-8 is a straight type infusion set, cannula 210 may be used in both angled and straight type insertion infusions sets. It may be advantageous due to the design of the particular infusion set being used with cannula 210 to predetermine the final shape of cannula 210 external to the patient 50, allow cannula 210 to be reshaped during fabrication, and then allow cannula 210 return to its original formed shape for final permanent location once heated by the human body as shown in Fig. 8.

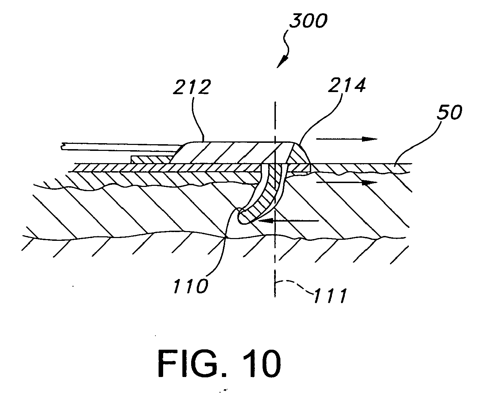

Figs. 9 and 10 show cannula 110 from Figs. 1a-1c used with the housing 212 and the base 214 from Figs. 6-8. The resulting infusion set 300 provides the insertion method of infusion set 200 with the superelasticity in cannula 110 as described with infusion set 100. Cannula 110 is free to flex away from its longitudinal axis 111 as shown generally in Fig. 9. Fig. 10 shows an application of the flexibility of cannula 110, with housing 212 and base 214 of the infusion set 300 being displaced from left to right as shown in the figure, with cannula 110 flexing to the left of longitudinal axis 111.

Since, in any configuration, the Nitinol cannula will not typically kink or pinch, patient safety is greatly increased, and diminished or blocked flow of medications will not occur. Since the material is strong and durable, the Nitinol cannula can also have very thin walls, and therefore smaller diameter, compared to stainless steel and polymer and is very flexible, thus patient comfort is also greatly improved.

Since Nitinol is a metallic material, the reliable application and adhesion of bio-coatings tuned for specific biological purposes is possible. This is not the case for most polymer cannulae such as PTFE (Teflon®), which is very hard to reliably coat. Fig. 8 shows the cannula 210 having been coated with a bio-coating 220. Such a bio-coating may be an anti-thrombotic, an anti-inflammatory, or an anti-microbial coating. In addition, since Nitinol is a highly biocompatible material, any adverse tissue reactions caused by the use of polymers would be eliminated.

Other significant advantages of a device with a Nitinol shape memory or superelastic cannula include the ability to assemble the device using standard adhesives and other processes well adapted to metallic parts. For example, Nitinol can be used with adhesives, such as cyanoacrylates and epoxies. Additionally, Nitinol can be soldered to other metals. Further, Nitinol can be sterilized using many common sterilization methods that cannot be utilized with traditional polymer cannulae. Still further, a Nitinol cannula can be sterilized using Gamma radiation, which cannot be used with PTFE cannulae because the radiation degrades the polymer significantly.

It should be noted the above-referenced descriptions of the infusion sets 100, 200, 300 with a Nitinol cannula assembly illustrate only desired designs of infusion sets 100, 200, 300. Many variations of shape, material configuration, and configuration of the infusion sets 100, 200, 300 and cannulae 110, 210 are possible within the scope of the invention. These design concepts may also be applied to I-V catheter designs for other medical applications.

Although Nitinol is one material discussed in this invention, those skilled in the art will recognize that any material with properties similar to Nitinol could be utilized. For example, superelastic alloys constructed from Cu-Al-Ni, Cu-Al-Zn, and Cu-Zn are also contemplated by this invention.

Although the invention is illustrated and described herein with reference to specific embodiments, the invention is not intended to be limited to the details shown. Rather, various modifications may be made in the details within the scope and range of equivalents of the claims and without departing from the invention.

## Claims

1. A cannula housing assembly for an infusion set comprising:
a cannula housing; and
a cannula extending distally from the cannula housing, wherein the cannula is constructed from one of a superelastic material and a shape memory material.

2. The cannula housing according to claim 1, wherein the cannula has a first shape at a first temperature and a second shape at a second temperature.

3. The cannula housing assembly according to claim 2, wherein the cannula is formed in the first shape at the first temperature and subsequently cooled to the second temperature.

4. The cannula housing assembly according to claim 1, wherein the cannula comprises a coating on at least a portion of the cannula.

5. The cannula housing assembly according to claim 1, wherein the cannula comprises a nickel-titanium alloy.

6. The cannula housing assembly according to claim 1, wherein the cannula comprises a flexible distal tip.

7. The cannula housing assembly according to claim 1, further comprising an insertion needle co-axially inserted through the cannula.

8. The cannula housing assembly according to claim 1, wherein the cannula comprises a cannula wall having a thickness of between about 0.02 and 0.03 mm.

9. The cannula housing assembly according to claim 1, further comprising a base, wherein the cannula housing is pivotally coupled to the base between an insertion position and a use position.

10. The cannula housing assembly according to claim 9, wherein the cannula has a first shape when the cannula housing is in the insertion position and a second shape when the cannula housing is in the use position.

11. A cannula housing assembly for an infusion set comprising:
a base;
a cannula housing coupled to the base; and
a cannula extending distally from the cannula housing, wherein the cannula is constructed from one of a superelastic material and a shape memory material.

12. The cannula housing according to claim 1, wherein the housing is pivotally coupled to the base between an insertion position and a use position.

13. The cannula housing according to claim 11, wherein the cannula has a first shape when the base is in the insertion position and a second shape when the base is in the use position.

14. A method of inserting a cannula into a patient comprising:
providing a cannula formed from a shape memory material;
forming the cannula into a desired shape;
reforming the cannula into an insertion shape;
inserting the cannula into the patient; and
allowing body heat from the patient to revert the cannula from the insertion shape to the desired shape.

15. The method according to claim 14, wherein providing the cannula from the shape memory material comprises providing the cannula from a metallic alloy.

16. The method according to claim 14, wherein providing the cannula comprises providing the cannula having a proximal end, a distal end, and a longitudinal axis extending therebetween and wherein allowing body heat from the patient to revert the cannula from the insertion shape to the desired shape comprises displacing at least one of the proximal end and the distal end away from the longitudinal axis.

17. The method according to claim 14, wherein forming the cannula comprises forming the cannula at a first temperature.

18. The method according to claim 17, wherein reforming the cannula comprises reforming the cannula at a second temperature, lower than the first temperature.

19. The method according to claim 18, wherein allowing body heat from the patient to revert the cannula comprises heating the cannula to a temperature higher than the first temperature.

20. The method according to claim 14, wherein the cannula is part of a cannula housing assembly, the method further comprising, after allowing body heat from the patient to revert the cannula from the insertion shape to the desired shape, moving the cannula housing assembly from an insertion position to a use position.
